# EUROPEAN PATENT SPECIFICATION

(11) **EP 2 179 046 B1**
(45) Date of publication and mention of the grant of the patent: **02.01.2013**
(21) Application number: 07790064.5
(22) Date of filing: 13.08.2007
(51) Int. Cl.: C12P 7/64, A23K 1/00, A23K 1/16, A23L 1/30

(54) **OVER-PRODUCTION OF DIHOMO GAMMA LINOLENIC ACID BY A MUTANT STRAIN OF PARIETOCHLORIS INCISA**
ÜBERPRODUKTION VON DIHOMO-GAMMA-LINOLENSÄURE DURCH EINEN PARIETOCHLORIS INCISA-MUTANTENSTAMM
SURPRODUCTION D'ACIDE DIHOMO-GAMMA-LINOLÉNIQUE PAR UNE SOUCHE MUTANTE DE PARIETOCHLORIS INCISA

(43) Date of publication of application: 28.04.2010
(73) Proprietor: Ben-Gurion University of the Negev Research and Development Authority, 84105 Beer-sheva (IL)
(72) Inventor: COHEN, Zvi, 84965 Omer (IL); KHOZIN-GOLDBERG, Inna, 84990 Midreshet Sde Boker (IL); BOUSSIBA, Sammy, 84965 Omer (IL); VONSHAK, Avigad, 84990 Midreshet Sde Boker (IL)
(74) Representative: Vossius & Partner
(86) International application number: PCT/IL2007/001012
(87) International publication number: WO 2009/022323

(56) References cited:
- EP-A- 0 711 503
- EP-A1- 0 535 940
- EP-A2- 0 195 570
- WO-A-01/84961
- WO-A1-03/079810
- US-B2- 6 602 690
- BIGOGNO C ET AL: "Lipid and fatty acid composition of the green oleaginous alga Parietochloris incisa, the richest plant source of arachidonic acid" PHYTOCHEMISTRY, PERGAMON PRESS, GB, vol. 60, no. 5, July 2002 (2002-07), pages 497-503, XP004359481 ISSN: 0031-9422 cited in the application
- BIGOGNO C ET AL: "Biosynthesis of arachidonic acid in the oleaginous microalga Parietochloris incisa (Chlorophyceae): Radiolabeling studies" LIPIDS, vol. 37, no. 2, February 2002 (2002-02), pages 209-216, XP008084833 ISSN: 0024-4201
- JAREONKITMONGKOL SAEREE ET AL: "A novel DELTA-5-desaturase-defective mutant of Mortierella alpina 1S-4 and its dihomo-gamma-linolenic acid productivity" APPLIED AND ENVIRONMENTAL MICROBIOLOGY, vol. 59, no. 12, December 1993 (1993-12), pages 4300-4304, XP002437027 ISSN: 0099-2240
- KAWASHIMA H ET AL: "Industrial production of dihomo-gamma-linolenic acid by a DELTA5 desaturase-defective mutant of Mortierella alpina 1S-4 fungus" JOURNAL OF THE AMERICAN OIL CHEMISTS' SOCIETY, vol. 77, no. 11, November 2000 (2000-11), pages 1135-1138, XP008084822 ISSN: 0003-021X
- CERTIK M ET AL: "Desaturase-defective fungal mutants: useful tools for the regulation and overproduction of polyunsaturated fatty acids" TRENDS IN BIOTECHNOLOGY, vol. 16, no. 12, 1 December 1998 (1998-12-01), pages 500-505, XP004143810 ISSN: 0167-7799
- GUSCHINA ET AL: "Lipids and lipid metabolism in eukaryotic algae" PROGRESS IN LIPID RESEARCH, vol. 45, no. 2, March 2006 (2006-03), pages 160-186, XP005299269 ISSN: 0163-7827
- DYAL S D ET AL: "Implications for the use of Mortierella fungi in the industrial production of essential fatty acids" FOOD RESEARCH INTERNATIONAL, vol. 38, no. 4, May 2005 (2005-05), pages 445-467, XP004778909 ISSN: 0963-9969
- WARD ET AL: "Omega-3/6 fatty acids: Alternative sources of production" PROCESS BIOCHEMISTRY, vol. 40, no. 12, December 2005 (2005-12), pages 3627-3652, XP002454689 ISSN: 1359-5113
- DATABASE HTTP://CMSPROD.BGU.AC.IL [Online] 7 February 2008 (2008-02-07), PROF. ZVI HACOHEN ET AL: "Production of the Polyunsaturated Fatty AcidDihomo- -? Linolenic Acid (DGLA) by a Microalgal Strain" XP002479245 retrieved from HTTP://CMSPROD.BGU.AC.IL/NR/RDONLYRES/DB35 05F1-2D48-4E71-B43A-120952269433/46473/WEB SITEDGLA.PDF

## Description

### Field of the invention

The present invention relates to a Parierochloris incisa mutant strain having ATCC Accession No. PTA-8497 and a process for the production of dihomo-gamma-linolenic acid (DGLA) from said microalga. More specifically, the process includes the cultivation of said arachidonic acid-rich microalga which has lost or significantly reduced their ability for DELTA 5 desaturation.

### Background of the invention

DGLA is an industrially-important fatty acid that can be used for pharmaceutical and nutritional applications, in feed for aquaculture and animals and for enhancing their immunological systems thereby reducing their mortality and morbidity due to stress and diseases. DGLA, also known as 8,11,14-eicosatrienoic acid, is found only in microorganisms and always at low percentages. Usually, it serves as an intermediate in the biosynthesis of arachidonic acid (AA, 20:4ω6), the conversion of DGLA to AA being facilitated by the enzyme Δ5 desaturase.

Microalgae are a "green" and renewable source of biochemicals. They can be cultivated phototrophically or adapted, or engineered for growth under heterotrophic conditions.

The freshwater alga Parietochloris incisa is the richest plant source of the polyunsaturated fatty acid (PUFA) AA. Under conditions of nitrogen starvation, the content of AA can reach 21% of dry weight.

Plant oils are capable of producing various PUFAs. However, those produced by higher plants are restricted to chains of up to 18 carbon atoms. Microalgae on the other hand, are known to produce PUFA of up to 22 carbon atoms long.

Both AA and DGLA are PUFA of significant pharmacological interest. DGLA is found in many organisms as an intermediate; however, there are few natural host organisms in which there is accumulation of significant amounts of this PUFA. Possibly the only such source is a Δ5 desaturase deficient mutant of the fungus *Mortierella* (US 6, 602, 690) . However, one drawback associated with the PUFAs produced by this fungal mutant is the unfavorably low DGLA/AA ratio (approximately 12). A further disadvantage of the fungal-derived PUFAs is that they are susceptible to oxidation and synthetic antioxidants need to be added to prevent deterioration by oxidation. Since the oxidation is a chain reaction, even a small amount of oxygen can destroy PUFA rapidly.

A need thus exists for a natural, "green" source of DGLA that overcomes the aforementioned problems.

### Summary of the invention

The present invention provides a mutant strain of *Parietochloris incisa*, characterized in comprising a substantially reduced intracellular concentration of arachidonic acid (AA) together with a substantially increased intracellular concentration of dihomo-gamma-linolenic acid 20:3ω6 (DGLA), wherein said strain is strain IKG-1, having ATCC Accession No. PTA-8497. Also provided is a process for producing DGLA, comprising incubating the mutant strain of the microalga *Parietochloris incisa* that is defective in its Δ5 desaturase (Δ5D) gene, and recovering DGLA-containing lipids therefrom. In a preferred process of the invention, the ratio of DGLA to AA (on a weight to weight basis) in the final product (i.e., in the DGLA-containing lipids) is greater than 25. In a particularly preferred embodiment, this ratio is greater than 50.

As a general guide, the phrase "substantially reduced intracellular concentration of AA", as used in the present context should be taken to mean that said concentration is about 1% of total fatty acids or less. Similarly, the phrase "substantially increased intracellular concentration of DGLA" refers to a DGLA concentration greater than about 30% of total fatty acids.

Envisaged is that, the intracellular concentration of AA is less than 0.1% (w/w), while the intracellular concentration of DGLA is at least 30% (w/w). Both of the aforementioned w/w % concentrations are to be understood as referring to the percentage of the indicated substance in relation to total cellular fatty acids.

The aforementioned mutant *Parietochloris incisa* strain is a strain which is defective in its Δ5 desaturase (Δ5D) gene. The term "defective" is used in this context to refer to the fact that the Δ5 desaturase (Δ5D) gene is either absent or has substantially less desaturase activity than the wild-type gene due to a point mutation (e.g. a substitution, insertion or deletion of a single nucleic acid base) or a larger mutation (e.g. an inversion or rearrangement of a sequence).

The present invention is particularly directed to *Parietochloris incisa* mutant strain IKG-1, which was deposited with Accession No. PTA-8497 at the American Type Culture Collection (ATCC), Manassas, Virginia, USA, under the terms of the Budapest Treaty.

Described is a DGLA-containing lipid, wherein the ratio of DGLA to arachidonic acid is at least 25 parts by weight. In another aspect described, the concentration of DGLA in said DGLA-containing lipid is at least 30% (w/w) or at least 90% (w/w) of the total lipids in said DGLA-containing lipid are triacylgycerols.

Further described is a prostaglandin E1 precursor of algal origin. Said prostaglandin E1 precursor can be obtained from the mutant strain of *Parietochloris incisa,* as characterized hereinabove.

It is to be noted that the term "prostaglandin E1 precursor" is used in this context to refer to any fatty acid or mixture of fatty acids that can serve as a starting material or intermediate in the synthesis of AA. Examples of such fatty acids include GLA and DGLA."

Described are compositions comprising DGLA-containing lipids and/or prostaglandin E1 precursors, all of which are characterized as described hereinabove. Such compositions may be prepared for use in a variety of applications including animal feeds, human infant formulations and food supplements.

For example,
the composition can be a health food composition comprising a DGLA-containing lipid referred to herein.
Said health food composition may be used, *inter alia*, for alleviating the side effects experienced following alcohol ingestion.

### Detailed description

In order to generate mutant strains of *P. incisa*, the wild-type strain may be exposed to a mutagenic agent and then plated on a solid medium at a low temperature. Colonies that demonstrate decreased growth under these conditions may then be selected and analyzed for their AA and DGLA content.

Cultures of *P*. *incisa* may be mutagenized by a variety of different techniques including irradiation (e.g. with X-ray or UV light), treatment with chemical mutagens (e.g. 1-methyl-3-nitro-nitrosoguanidine, methanesulfonate and 6-mercaptopurine.

Following mutagenesis, the cells are plated on agar plates, and brought to a low temperature (e.g. 15 °C). Colonies that showed decreased growth can then be isolated and analyzed for lipid content.

The present inventors unexpectedly found that one of the colonies selected as described above proved to be deficient in AA. Under nitrogen starvation conditions the proportion of AA in this mutant was lower than 1% in comparison to over 50% in the wild type. However, the proportion of AA's immediate precursor, DGLA (20:3ω6) increased from about 1% in the wild type to over 30% in the mutant. The DGLA/AA ratio in the PUFAs produced by the algal mutant is greater than 25, and in certain circumstances, greater than 50, both of which ratios are clearly superior to the DGLA/AA ratio of 12 associated with PUFAs produced from the fungal mutant described in the aforementioned prior art.

In view of the aforementioned altered DGLA/AA ratio, it may be concluded that the selected mutant strain is defective in its Δ5 desaturase (Δ5D) gene that desaturates DGLA to AA. Similar to the wild type, the mutant is capable of accumulating high contents of lipids, mostly triacylglycerols, under nitrogen starvation, enabling the biotechnological application of the process.

DGLA-rich oil of *P. incisa* mutant is superior to the fungal oil in that it contains P carotene, a natural antioxidant. Fungal PUFAs are susceptible to oxidation and synthetic antioxidants need to be added to prevent deterioration by oxidation. Since the oxidation is a chain reaction, even a small amount of oxygen can destroy PUFA rapidly.

As mentioned hereinabove, described are compositions comprising DGLA-containing lipids and/or prostaglandin E1 precursors.
Such compositions may be prepared for use in a variety of applications including, but not limited to, animal feeds, human infant formulations, food supplements and health food composition's. In addition to the aforementioned DGLA-containing lipids, and/or prostaglandin E1 precursors, said compositions may additionally comprise further active components including pharmaceutically-active and nutraceutically-active agents, as well as fillers, carriers, buffers, bulking agents and other excipients and inactive ingredients as are well known in the art. Further details of excipients that may be used in pharmaceutical and nutraceutical compositions may be found in standard reference works such as Remington's Pharmaceutical Sciences, Mack Publishing Co, Easton, Pa, USA (1980).

The following example is provided for illustrative purposes and in order to more particularly explain and describe the present invention.

### Example

### Mutagenesis

The *P. incisa* strain was isolated from a snow sample of Mt. Oyama (Japan). The microalga has been identified as the chlorophyte, *Parietochloris incisa* (Trebouxiophyceae) (Watanabe et al. 1996, Phycol. Res. 44: 107-8).

Prior to mutagenesis, cultures were cultivated in BG-11 nutrient medium (Stanier et al., 1971, Bacteriological Reviews, 35: 171-205) in 150 mL flasks under controlled temperature and light conditions. The flasks were placed in a temperature regulated shaker at 25 °C and illuminated by cool white fluorescent lights at a light intensity of 115 µmol photon m⁻²s⁻¹ as previously described (Bigogno et al. 2002, Phytochemistry 60, 497-503). The cultures were provided with a mixture of air and CO₂ (99:1, v/v).

Fifty mL of logarithmically growing cultures were withdrawn and sonicated in 150 mL flasks to segregate the cell clumps. Ten mL of cell suspension, containing mostly single cells, were exposed to the mutagen, 1-methyl-3-nitro-nitrosoguanidine (MNNG) at a final concentration of 100 µg/mL. The stock solution of MNNG was prepared in DMSO (5 mg/mL) to ease the penetration of the mutagen across the tough cell wall of the alga. Following 1 h incubation in an incubator shaker, cells were harvested by centrifugation (1500 x g) and washed with mutagen-free BG-11 medium. The washing step was repeated several times. Finally, cells were sonicated in 10 mL of medium and cell numbers of untreated and treated cultures were counted. Cell suspensions were sequentially diluted up to 1000 cells/mL and 50-100 cells were plated onto Petri dishes with BG-11 agar medium. The resulting cultures were duplicated and incubated under fluorescent light at room and low temperature (15°C). The percent of survived cells was determined after 10 days. Colonies, which showed poor growth or different appearance at low temperature, were selected for cultivation on liquid BG11 medium in flasks. After sufficient biomass was achieved, cells were transferred onto liquid nitrogen free medium for 14 days, harvested and analyzed for fatty acid composition.

The fatty acid composition of the wild type (WT) and Δ5 desaturase-deficient mutant (MUT) of *P*. *incisa,* expressed as a wt/wt percentage of total fatty acids is shown in the following table.

| Strain | Fatty acid composition (% of total FA) | | | | | | | | | | DGLA AA | TFA (%DW) |
|---|---|---|---|---|---|---|---|---|---|---|---|---|
| | 16:0 | 16:2 | 18:0 | 18:1 | 18:1 | 18:2 | 18:3 | 18:3 | 20:3 | 20:4 | | |
| | | ω6 | | ω9 | ω7 | | ω3 | ω6 | ω6 | ω6 | | |
| WT | 10.9 | 0.6 | 2.8 | 11.0 | 4.3 | 11.1 | 0.9 | 0.9 | 1.2 | 54.6 | 0.02 | 33.3 |
| MUT | 8.0 | 0.2 | 1.9 | 34.4 | 2.9 | 12.8 | 1.2 | 1.1 | 34.4 | 0.1 | 340 | 36.5 |

## Claims

1. A mutant strain of *Parietochloris incisa,* **characterized in** comprising a substantially reduced intracellular concentration of arachidonic acid (AA) together with a substantially increased intracellular concentration of dihomo-gamma-linolenic acid 20:3ω6 (DGLA), wherein said strain is strain IKG-1, having ATCC Accession No. PTA-8497.

2. A process for producing DGLA, comprising incubating a mutant strain of the microalga *Parietochloris incisa* that is defective in its Δ5 desaturase (Δ5D) gene, and recovering DGLA-containing lipids therefrom, wherein said strain is strain IKG-1, having ATCC Accession No. PTA-8497.

3. The process according to claim 2, wherein the ratio of DGLA to AA in the DGLA-containing lipids is greater than 25:1 by weight.

## Patentansprüche

1. *Parietochloris incisa*-Mutantenstamm, **dadurch gekennzeichnet, dass** er eine substantiell reduzierte intrazelluläre Konzentration von Arachidonsäure (AA) zusammen mit einer substantiell erhöhten intrazellulären Konzentration von Dihomo-gamma-Linolensäure 20:3ω6 (DGLA) umfasst, wobei der Stamm der IKG-1-Stamm mit der ATCC-Zugangsnummer PTA-8497 ist.

2. Verfahren zur Herstellung von DGLA, umfassend das Inkubieren eines Mutantenstamms der Mikroalge *Parietochloris incisa*, der defizient in seinem Δ5 Desaturase (Δ5D)-Gen ist, und das Gewinnen der DGLA-enthaltenden Lipide daraus, wobei der Stamm der IKG-1-Stamm mit der ATCC-Zugangsnummer PTA-8497 ist.

3. Verfahren nach Anspruch 2, wobei das Verhältnis von DGLA zu AA in den DGLA-enthaltenden Lipiden größer als 25:1 bezogen auf das Gewicht ist.

## Revendications

1. Souche mutante de *Parietochloris incisa*, **caractérisée en ce qu'**elle comprend une concentration intracellulaire d'acide arachidonique (AA) substantiellement réduite ainsi qu'une concentration intracellulaire d'acide dihomo-gama-linolénique 20:3ω6 (DGLA) substantiellement augmentée, dans laquelle ladite souche est la souche IKG-1 ayant le numéro d'entrée ATCC PTA-8497.

2. Procédé pour produire du DGLA, comprenant l'incubation d'une souche mutante de la micro-algue *Parietochloris incisa* qui est défectueuse pour son gène de la Δ5 désaturase (Δ5D), et la récupération des lipides contenant le DGLA ainsi formés, dans lequel ladite souche est la souche IKG-1 ayant le numéro d'entrée ATCC PTA-8497.

3. Procédé selon la revendication 2, dans lequel le ratio DGLA sur AA dans les lipides contenant le DGLA est supérieur à 25:1 en poids.
